# EUROPEAN PATENT SPECIFICATION

(11) **EP 1 313 517 B1**
(45) Date of publication and mention of the grant of the patent: **17.12.2008**
(21) Application number: 01949674.4
(22) Date of filing: 11.07.2001
(51) Int. Cl.: A61L 9/20, F24F 3/16

(54) **PURIFICATION OF AIR**
LUFTREINIGUNG
PURIFICATION D'AIR

(30) Priority: 11.07.2000 GB 0017058
(43) Date of publication of application: 28.05.2003
(73) Proprietor: Microgenix Australasia Pty Ltd, Sydney, NSW 2000 (AU)
(72) Inventor: HALL, Philip, c/o Microgenix Limited, Crayford, Kent DA1 4TF (GB)
(74) Representative: Crawford, Andrew Birkby
(86) International application number: PCT/GB2001/003124
(87) International publication number: WO 2002/004036

(56) References cited:
- EP-A- 0 673 656
- WO-A-92/20974
- GB-A- 2 212 370
- GB-A- 2 215 234
- US-A- 3 757 495
- US-A- 3 846 072
- US-A- 5 112 370
- US-A- 5 225 167

## Description

The present invention relates to a method and apparatus for purifying air.

The purification of air is a long standing problem and the problems are increased when recirculated air is being utilised as occurs in many enclosed spaces such as ships, vehicles, offices, aircraft and hospitals. There have been many attempts to improve the purification of air generally and one such system is disclosed in US Patent No 4,017,736 which discloses an air purification system utilising a pre-filter metal mesh and sub-micron laminar flow cell to remove particles and high intensity ultraviolet generator to kill microorganisms as air is forced by a fan though a shielded enclosure. This previous arrangement is designed to a free standing unit in an open area, and is not disclosed as being suitable for use with recirculated air.

There are particular problems with purifying air for recirculation in enclosed spaces and particularly in ships, offices aircraft and hospitals. Up to now, filtering the air has been the preferred method of air purification but this has been shown to be either inadequate or else energy inefficient.

EP 0,673,656 A discloses an air purification system including passing air through a filter carrying a disinfectant, and subsequently through a UV radiation stage.

WO 92/20974 A1 discloses an air purification system including a first baffle for reducing the turbulence of an air stream and ultraviolet lamps to irradiate the air stream to disinfect it.

GB 2,212,370 A discloses an air purification system including vanes of partitions for guiding a flow of air within a casing containing ultraviolet radiation tubes.

From a first aspect the present invention provides a method of purifying air comprising withdrawing air from an enclosed space, passing the withdrawn air over surfaces coated with an antimicrobial agent, through an ultraviolet radiation and returning the thus irradiated air to the enclosed space , characterised in that turbulence is caused to the air flow prior to passing the withdrawn air through the ultraviolet radiation.

From a second aspect the present invention provides an apparatus for purifying air comprising means for withdrawing air from an enclosed space, ducting for directing withdrawn air through a unit including an ultraviolet radiation section for irradiating the withdrawn air and thence back to an air inlet arranged to communicate with the enclosed space, the unit having at least one of its internal surfaces coated with an antimicrobial agent, the unit having at least one of its internal surfaces coated with an antimicrobial agent **characterised in that** the apparatus further comprising means for causing turbulence to the air flow prior to passing the withdrawn air through ultraviolet radiation section.

Preferably, the ultraviolet radiation emission section is arranged to be maintained in situ and is provide with one or more openable inspection panels provided with means which automatically terminate energisation of the ultraviolet light source upon opening the or each panel.

Additionally, it is preferred to control the ultraviolet light source in such a manner that ozone is not produced but, if necessary, ozone filters can be included.

In order that the present invention be more readily understood, an embodiment thereof will now be described with reference to the accompanying drawings, in which:-
Figure 1 shows a diagrammatic representation of an installation according to the present invention;
Figure 2 shows a diagrammatic side view of a portion of the installation shown in Figure 1;
Figure 3 shows a diagrammatic representation of a control box used in the installation shown in Figure 1.

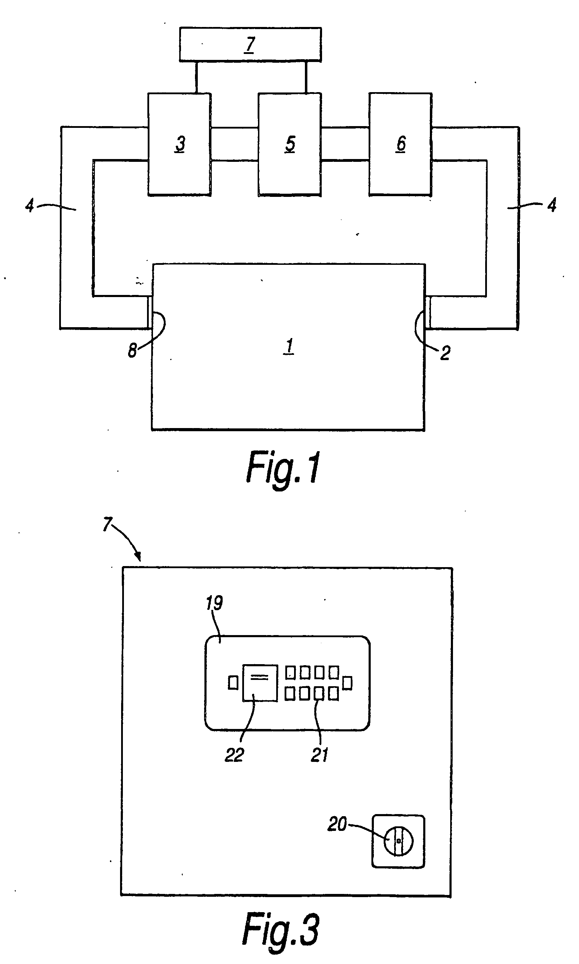

Referring now to Figure 1, this shows diagrammatically a recirculating air system for purifying air in an aircraft. This is but one application of the present invention and it is suitable for use in any situation where recirculated air is being utilised eg in a ship, motor vehicle, clean room or in offices and hospitals. The enclosed space 1 is provided with an air extraction vent 2 which permits air to be withdrawn from the enclosed space 1 under action of a fan system 3 via ducting 4. Located at a suitable position along the length of the ducting 4 is an ultraviolet illumination section 5 which will be described in detail below. Additionally, if required, an air flow conditioning section 6 is also provided in the ducting 4 which is arranged to provide conventional mechanical filtering of the air flowing through the ducting 4. The thus cleaned air is returned to the enclosed space via an air inlet 8 and the operation of the fan system 3 and the ultraviolet illumination section 5 is monitored and/or controlled by a control unit 7.

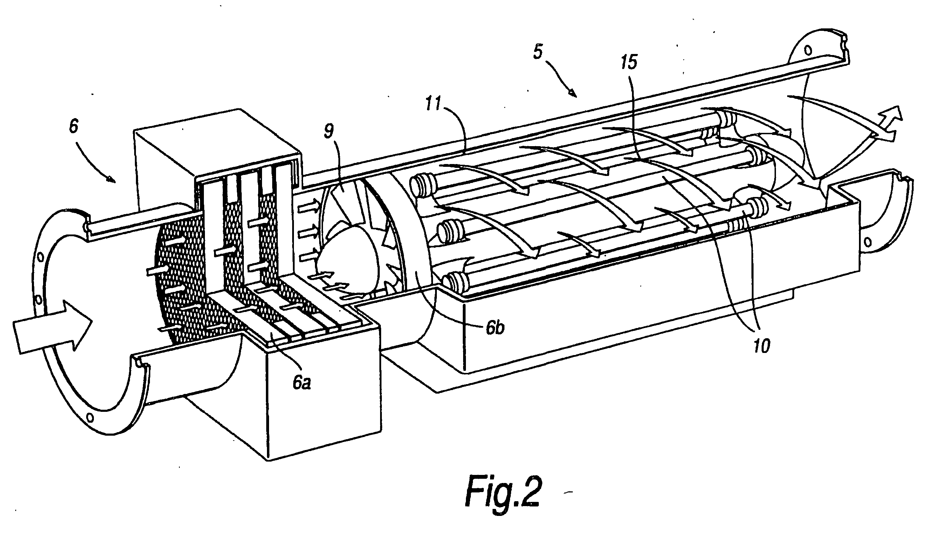

As mentioned above, air extracted from the enclosed space is drawn through the air flow conditioning section 6 which is shown in more detail in Figure 2 in combination with the ultraviolet illumination section 5. The section 6 can include one or more filter sections 6a and a pre-sterilisation section 6b. The filters used can be one or more of the conventional mechanical filters such as an activated charcoal filter as well as an electrostatic filter. It is important that the filters do not create a back pressure of any significance in the system and we have found a filter of 5 microns (G4/EU4 rating) to be sufficient. The pre-sterilisation section 6b includes an arrangement for creating slightly turbulent air flow through the ultraviolet illumination section 5. This can be achieved in a number of ways but we prefer to utilise a fixed multi bladed directional fan 9. Alternatively, the fan may be replaced by fixed shaped or planar members may be provided at an angle to the air flow in order to provide a sinous air flow path through the section 5. The fixed shaped or planar members may be perforated or made of a porous material.

The ultraviolet illumination section 5 will now be described and is shown as being of circular cross section but it will be appreciated that any convenient cross section may be utilised which may or may not be the same cross section as used for the ducting 4. The section 5 has a cylindrical side wall 11 which encloses a central core 15 around which are mounted the individual light sources 10. The number and length of the sources 10 will depend on the air flow conditions but in the present embodiment we prefer to have eight light sources 10 uniformly disposed around the core 15 and the effect of this is to cause slightly turbulent air flow in the annular space between the core 15 and the cylindrical side wall 11. The light sources 10 are held in position such that they are spaced from the core 15 which maximises the effect of the ultraviolet light on the bacteria in the air as the air flow passes over substantially all the surface of the sources 10.

The surfaces of the wall 11 and the central core 15 are either made of or coated with material with a high reflectance to UV light. We prefer to use polished aluminium sheet which has been found to be better than polished stainless steel. The side walls 11, can be provided with an openable panel to permit maintenance of the light sources 10 and permit other access to the illumination section 5. The individual light sources 10 are attached to the core 15 in any convenient manner and are most conveniently attached by means of simple clips. Additionally, the panel in the side wall 11 may be provided with detectors (not shown) which will detect the opening or potential opening of a panel and thus signal to the control unit 7 that the light sources 10 should be switched off. This avoids possible eye damage to maintenance personnel. The exact way in which the potential opening of the panels is signalled to the control unit is a matter of design choice and could be a micro switch or other suitable sensor which could be activated by the operation of a latch or the like on the side wall which is required to be unlatched prior to the movement of the panel itself.

Figure 3 shows a typical front panel 19 for the control unit 7 where an isolator switch 20 is shown. The control unit can be located at any convenient position but will usually be remote from the fan system 3 and illumination section 5. A key pad 21 can provide control of the illumination section 5 and also of fan speed to cater for different conditions and an LFI and hours run meter 22 is also provided which can be checked by maintenance personnel.

The proposed method is to use the ultraviolet light within the illumination section 5 in such a manner as to provide an excess of a 99% sanitation rate overall. It is also proposed to recirculate the air at regular intervals in order to improve the sanitation rate.

We have also found it to be advantageous to coat the internal surfaces of the pre-sterilisation section 6b and/or the filter section 6a with an antimicrobial agent. Preferably the agent is one which is non-leaching and nonvolatile and is not consumed by microorganisms. One suitable agent is a standard antimicrobial substance (a quaternary amine) in a silane which when coated on a surface bonds to the surface to render it antimicrobially active. In particular, an agent incorporating 3-(trimethoxysilyl)-propyldimethyloctadecyl ammonium chloride as active ingredient as sold by Aegis Environments of Midland, Michigan, USA under the trademark Aegis Microbe Shield is particularly useful.

Using an antimicrobial agent, it is preferred to use a metal "wool" as the filter in the filter section 6a and this is usually of stainless steel or other non-corroding metal. However, other filter materials such as natural or synthetic fibres or mixtures thereof could be used.

Alternatively, or in addition, we have found it advantageous to coat the internal surfaces of the ultra-violet illumination section with the microbial agent.

Although the apparatus described above can be assembled from discrete parts or modules, it is preferred to construct the conditioning section 6 and the UV treatment section 5 as a unit so that the unit can be readily fitted into existing ducting.

By using a three stage purification process, filtration, antimicrobial treatment and UV treatment it is possible to readily achieve a sanitation rate of in excess of 99% when recirculating the air.

## Claims

1. A method of purifying air comprising withdrawing air from an enclosed space (1), passing the withdrawn air over surfaces coated with an antimicrobial agent, through an ultraviolet radiation (5,10) and returning the thus irradiated air to the enclosed space (1), **characterised in that** turbulence (6b) is caused to the air flow prior to passing the withdrawn air through the ultraviolet radiation (5,10).

2. A method according to claim 1, comprising filtering (6a) the withdrawn air.

3. A method according to claim 1 or claim 2, wherein the agent is an antimicrobial substance in a silane.

4. A method according to any of claims 1 to 3, comprising detecting the opening or potential opening of a panel which permits access to an ultraviolet radiation light source (5,10), and signalling a control unit (7) that the light source (5,10) should be switched off.

5. Apparatus for purifying air comprising means (3) for withdrawing air from an enclosed space (1), ducting (4) for directing withdrawn air through a unit including an ultraviolet radiation section (5,10) for irradiating the withdrawn air and thence back to an air inlet (8) arranged to communicate with the enclosed space (1), the unit having at least one of its internal surfaces coated with an antimicrobial agent, **characterised in that** the apparatus further comprising means (6b) for causing turbulence to the air flow prior to passing the withdrawn air through ultraviolet radiation section (5,10).

6. Apparatus according to claim 5, comprising a filter (6a) for filtering the withdrawn air.

7. Apparatus according to claim 5 or claim 6, wherein the antimicrobial agent is coated on at least some of the internal surfaces of the filter (6a) and/or the air flow separating means (6b).

8. Apparatus according to any of claims 5 to 7, wherein the agent is an antimicrobial substance in a silane.

9. Apparatus according to any of claims 5 to 8, comprising a panel that is operable for providing access into the ultraviolet radiation section (5), the panel being provided with detectors for detecting the opening or potential opening of the panel and for signalling a control unit (7) that the light source (5,10) should be switched off.

10. Apparatus according to any of claims 5 to 9, wherein the means (6b) for causing turbulence comprises a fixed multi bladed directional fan (9).

## Patentansprüche

1. Verfahren zur Reinigung von Luft, umfassend Abziehen von Luft aus einem geschlossenen Raum (1), Leiten der abgezogenen Luft über Oberflächen, die mit einem antimikrobiellen Mittel beschichtet sind, durch eine ultraviolette Strahlung (5, 10) und Rückführen der auf diese Weise bestrahlten Luft zu dem geschlossenen Raum (1), **dadurch gekennzeichnet, dass** vor dem Durchleiten der abgezogenen Luft durch die ultraviolette Strahlung (5, 10) die Luft einer Durchwirbelung (6b) ausgesetzt wird.

2. Verfahren nach Anspruch 1, umfassend ein Filtern (6a) der abgezogenen Luft.

3. Verfahren nach Anspruch 1 oder Anspruch 2, wobei das Mittel eine antimikrobielle Substanz in einem Silan ist.

4. Verfahren nach einem der Ansprüche 1 bis 3, umfassend ein Erfassen der Öffnung oder möglichen Öffnung einer Verkleidung, die einen Zugang zu einer Lichtquelle mit UV-Strahlung (5, 10) ermöglicht, und Signalisieren einer Steuereinheit (7), dass die Lichtquelle (5, 10) ausgeschalten werden sollte.

5. Vorrichtung zur Reinigung von Luft, umfassend Mittel (3) zum Abziehen von Luft aus einem geschlossenen Raum (1), Rohrleitung (4) zum Leiten der abgezogenen Luft durch eine Einheit, die einen Abschnitt zur ultravioletten Bestrahlung (5, 10) zur Bestrahlung der abgezogenen Luft einschließt, und von dort zurück zu einem Lufteinlass (8), der für eine Verbindung mit dem geschlossenen Raum (1) angeordnet ist, wobei mindestens eine der Innenoberflächen der Einheit mit einem antimikrobiellen Mittel beschichtet ist, **dadurch gekennzeichnet, dass** die Vorrichtung ferner Mittel (6b) zum Bewirken einer Durchwirbelung des Luftstroms vor dem Durchleiten der abgezogenen Luft durch den Abschnitt zur ultravioletten Bestrahlung (5, 10) umfasst.

6. Vorrichtung nach Anspruch 5, umfassend einen Filter (6a) zum Filtern der abgezogenen Luft.

7. Vorrichtung nach Anspruch 5 oder Anspruch 6, wobei das antimikrobielle Mittel auf mindestens einige der Innenoberflächen des Filters (6a) und/oder des Mittels (6b) zum Auftrennen des Luftstroms beschichtet ist.

8. Vorrichtung nach einem der Ansprüche 5 bis 7, wobei das Mittel eine antimikrobielle Substanz in einem Silan ist.

9. Vorrichtung nach einem der Ansprüche 5 bis 8, umfassend eine Verkleidung, die bedienbar ist, um einen Zugang in den Abschnitt zur ultravioletten Bestrahlung (5) bereitzustellen, wobei die Verkleidung mit Detektoren zum Erfassen der Öffnung oder möglichen Öffnung der Verkleidung und zum Signalisieren einer Steuereinheit (7), dass die Lichtquelle (5, 10) ausgeschalten werden sollte, bereitgestellt wird.

10. Vorrichtung nach einem der Ansprüche 5 bis 9, wobei das Mittel (6b) zum Bewirken einer Durchwirbelung ein feststehendes Mehrschaufelrichtgebläse (9) umfasst.

## Revendications

1. Procédé pour purifier l'air comprenant les étapes consistant à aspirer l'air d'un espace clos (1), faire passer l'air aspiré sur des surfaces recouvertes avec un agent antimicrobien, par un rayonnement ultraviolet (5, 10) et faire revenir l'air ainsi rayonné dans l'espace clos (1), **caractérisé en ce que** l'on provoque une turbulence (6b) pour faire s'écouler l'air avant de faire passer l'air aspiré par le rayonnement ultraviolet (5, 10).

2. Procédé selon la revendication 1, comprenant l'étape consistant à filtrer (6a) l'air aspiré.

3. Procédé selon la revendication 1 ou la revendication 2, dans lequel l'agent est une substance antimicrobienne dans un silane.

4. Procédé selon l'une quelconque des revendications 1 à 3, comprenant les étapes consistant à détecter l'ouverture ou l'ouverture potentielle d'un panneau qui permet l'accès à une source lumineuse de rayonnement ultraviolet (5, 10), et signaler à une unité de commande (7) que la source lumineuse (5, 10) doit être arrêtée.

5. Appareil pour purifier l'air comprenant des moyens (3) pour aspirer l'air d'un espace clos (1), une conduite (4) pour diriger l'air aspiré dans une unité comprenant une section de rayonnement ultraviolet (5, 10) pour rayonner l'air aspiré et le ramener ainsi jusqu'à une entrée d'air (8) agencée pour communiquer avec l'espace clos (1), l'unité ayant au moins l'une de ses surfaces internes recouverte avec un agent antimicrobien, **caractérisé en ce que** l'appareil comprend en outre des moyens (6b) pour provoquer une turbulence afin que l'air s'écoule avant de faire passer l'air aspiré dans la section de rayonnement ultraviolet (5, 10)).

6. Appareil selon la revendication 5, comprenant un filtre (6a) pour filtrer l'air aspiré.

7. Appareil selon la revendication 5 ou la revendication 6, dans lequel l'agent antimicrobien est appliqué sur au moins certaines des surfaces internes du filtre (6a) et/ou les moyens de séparation d'écoulement d'air (6b).

8. Appareil selon l'une quelconque des revendications 5 à 7, dans lequel l'agent est une substance antimicrobienne dans un silane.

9. Appareil selon l'une quelconque des revendications 5 à 8, comprenant un panneau qui fonctionne pour fournir l'accès à la section de rayonnement ultraviolet (5), le panneau étant prévu avec des détecteurs pour détecter l'ouverture ou l'ouverture potentielle du panneau et pour signaler à une unité de commande (7) que la source lumineuse (5, 10) doit être arrêtée.

10. Appareil selon l'une quelconque des revendications 5 à 9, dans lequel les moyens (6b) pour provoquer la turbulence comprennent un ventilateur directionnel (9) à plusieurs pales fixes.
